# EUROPEAN PATENT SPECIFICATION

(11) **EP 2 037 757 B1**
(45) Date of publication and mention of the grant of the patent: **19.06.2013**
(21) Application number: 07719895.0
(22) Date of filing: 01.06.2007
(51) Int. Cl.: A23K 1/16, A23C 15/00, A23C 19/00, A23C 9/00, A23C 9/152, A23G 9/32, A23K 1/00, A23K 1/10, A23L 1/29, A23K 1/18, A61K 35/20, A23G 9/36, A23L 1/30, A23C 19/076

(54) **FEED PRODUCT FOR DAIRY COWS AND METHOD OF OBTAINING A DAIRY PRODUCT**
TIERFUTTERMITTEL UND METHODE ZUR HERSTELLUNG EINES MILCHPRODUKTS
ALIMENT POUR VACHES LAITIERES ET METHODE DE PREPARATION D'UN PRODUIT LAITIER

(30) Priority: 02.06.2006 US 445186
(43) Date of publication of application: 25.03.2009
(73) Proprietor: The University of Guelph, Guelph ON N1G 2W1 (CA)
(72) Inventor: EINO, Moni, Mississauga, Ontario L5L 2Y1 (CA); MILLIGAN, Larry, Guelph, Ontario N1H 6J2 (CA); OSBORNE, John, Elmira, Ontario N3B 3J5 (CA)
(74) Representative: Fleuchaus, Michael A.
(86) International application number: PCT/CA2007/000979
(87) International publication number: WO 2007/140589

(56) References cited:
- EP-A1- 0 588 707
- WO-A1-00/44239
- WO-A1-02/00028
- CA-A1- 2 444 189
- CA-A1- 2 524 451
- JP-A- 8 336 360
- US-A- 4 533 557
- US-A- 5 932 257
- US-A- 5 985 348
- US-A1- 2004 058 003
- US-B2- 7 063 855
- BORIS J ET AL: "A RANDOMIZED CONTROLLED TRIAL OF THE EFFECT OF FISH OIL SUPPLEMENTATION IN LATE PREGNANCY AND EARLY LACTATION ON THE N-3 FATTY ACID CONTENT IN HUMAN BREAST MILK", LIPIDS, SPRINGER, US, vol. 39, no. 12, 1 December 2004 (2004-12-01), pages 1191-1196, XP009065060, ISSN: 0024-4201, DOI: 10.1007/S11745-004-1347-7
- WRIGHT T C ET AL: "Effect of combinations of fish meal and feather meal on milk fatty acid content and nitrogen utilization in dairy cows.", JOURNAL OF DAIRY SCIENCE, vol. 86, no. 3, 2003, pages 861-869, XP009156001, ISSN: 0022-0302
- FRANKLIN S T ET AL: "DIETARY MARINE ALGAE(SCHIZOCHYTRIUM SP.) INCREASES CONCENTRATIONS OF CONJUGATED LINOLEIC, DOCOSAHEXAENOIC AND TRANSVACCENIC ACIDS IN MILK OF DAIRY COWS", THE JOURNAL OF NUTRITION, WISTAR INSTITUTE OF ANATOMY AND BIOLOGY, vol. 129, no. 11, 1 November 1999 (1999-11-01), pages 2048-2054, XP000879209, ISSN: 0022-3166
- KAMMERLEHNER J: "VORKOMMEN VON OMEGA-3-FETTSAEUREN IM MILCHFETT - IHRE GESUNDHEITLICHE BEDEUTUNG", DEUTSCHE MILCHWIRTSCHAFT, HILDESHEIM, DE, vol. 46, no. 2, 1 January 1995 (1995-01-01), pages 68-70, XP002042828, ISSN: 0012-0480

## Description

### FIELD OF INVENTION

The present invention relates to feed products for dairy cows comprising a source of long chain omega-3 fatty acids (DHA, EPA and DPA). The invention also relates to a method for feeding dairy cattle to achieve enhanced long-chain omega-3 fatty acid levels in milk.

### BACKGROUND OF THE INVENTION

Long chain omega-3 fatty acids (LC n-3 FA) are important nutrients necessary for maintaining human health from conception to childhood and throughout life. Docosahexaenoic acid (DHA; C-22:6 n-3), an important LC n-3 FA, is a major component of nerve, brain and eye tissue. DHA has been associated with a variety of human health benefits including the prevention and management of cardiovascular disease in adults, improved visual acuity in infants, management of inflammatory disease in adults and help with attention deficit disorder. Other LC n-3 FA that have been shown to have beneficial physiological effects when consumed include eicosapentanoic acid (EPA; C-20:5 n-3) and docosapentanoic acid (DPA; C-22:5 n-3).

The diets of people in many countries and cultures have been shown to be deficient in LC n-3 FA. The American Heart Association recommends the daily consumption of fish or fish oil supplement for adults to achieve a combined DHA and EPA intake of about 900 mg/d. The current mean intake of DHA and EPA (combined) for adults in North America (which includes about one fish serving every 10 days) is approximately 130 mg/d or 14% of the target intake.

LC n-3 FA have been shown to be required nutrients for optimal maturation of visual and cortical function in human infants. Evidence suggests that breast fed infants have a long-term advantage in cognitive development over formula fed infants. There is some suggestion that the deficiency of LC n-3 FA such as DHA in infant formulas and cow's milk may be an important factor correlating with these observations.

It is therefore apparent that it would be desirable to add LC n-3 FA and/or their sources to ingestible formulations. However, several characteristics make their inclusion in good tasting food a challenge. LC n-3 FA such as DHA, EPA, and DPA are typically derived from a fatty fish and are often used in the form of fish oil with the attendant flavour and odour being major barriers to use. Additionally, these fatty acids containing multiple conjugated double bonds are extremely susceptible to oxidation and rancidity. Thus, their purification, concentration and storage is very difficult As a result, a DHA-containing fish oil, or oil from in vitro cultured algae, which is subjected to concentration, purification, and anti-oxidation is very costly to produce and this also limits their use.

Various types of nutritional supplements containing DHA have been developed. These are usually provided in a compressed tablet or capsule format. While these supplements do provide health benefits, they are still associated with a fishy flavor. An increasing area of interest is food products such as fat spreads like margarine, instant powder concentrates, liquid egg preparations and bread containing DHA. It has been found however that while food products with strong flavors and/or sweetness can significantly mask the unpleasant fishy taste, milder tasting products are unsuitable for this type of enrichment due to poor palatability. In addition, encapsulated oil tends to separate, particularly from liquid products and requires consumption of high levels of capsular material.

Bovine milk and dairy products play an important role in human health and nutrition. The Canada Food Guide recommends the daily consumption of dairy products for people of all ages. Consumption of milk is particularly important for children. In fact, milk is by far and away the primary source of fatty acids for children. Thus, it would be desirable to provide LC n-3 FA- enhanced dairy products for consumption from conception throughout life. However, addition of exogenous fish oil to milk results in a foul taste.

Several attempts have been made to provide milk-like products or other products that do contain LC n-3 FA.

United States Patent No. 6,727,373 discloses a microbial LC n-3 FA-containing oil with a high triglyceride content and a high oxidative stability. In addition, a method is described for the recovery of such oil from a microbial biomass derived from a pasteurized fermentation broth, wherein the microbial biomass is subjected to extrusion to form granular particles, dried and the oil then extracted from the dried granules using an appropriate solvent.

United States Patent No. 6,428,832 relates to a process for the preparation of a LC n-3 FA-containing food, such as an infant formula, where a composition comprising a LC n-3 FA is added at a late stage of the infant formula preparation process. In this way, the LC n-3 Fa's are minimally exposed to conditions during the process that induce their degradation.

United States Patent Application No. 2004/0131727 provides dairy products containing fish-oil originated EPA and/or DHA and having oxidation and emulsification stability. The soy is acidified by addition of an acid, fermented milk, or acidified milk containing any of the milk acidified by addition of an acid and the fermented milk. The acidified milk contains EPA and/or DHA as fish oil, preferably purified fish oil or fish oil containing EPA and/or DHA in adjusted amount. The acidified milk is produced through an emulsification process. Preferably, the emulsification is performed after a fermentation process of the acidified milk process through a two-stage emulsification process. A food product containing the acidified milk of the invention is also provided.

United States Patent No. 5,976,606 provides a process for producing a DHA-containing tofu or soybean milk drink, or a dry powder thereof that is stable and inexpensive. A DHA-containing fish oil emulsion is prepared by mixing soybean milk with a DHA-containing fish oil at a weight ratio of 1:0.2 to 1:1, and stirring the mixture. The soybean milk drink can be produced by further diluting this emulsion with soybean milk to give a predetermined DHA concentration. DHA-containing tofu can be produced by coagulating the soybean milk containing this emulsion with the addition of a coagulant. Since the same soybean milk as the starting material of tofu or soybean milk drink is used as an emulsifying agent, even if DHA is contained at a high concentration, this does not influence the taste or properties of the product. A dry powder can be obtained by freeze-drying, vacuum drying or spray-drying this tofu or soybean milk drink as required.

United States Patent Application No. 2004/0265462 provides an infant milk formula having long chain polyunsaturated fatty acids, sialic acids, and cholesterol.

United States Patent No. 6,596,302 provides methods for providing nutrition and for enhancing neurological development of preterm infants. Also disclosed is an improved nutritional composition containing specified amounts of DHA and arachidonic as well as their precursor (in some species) fatty acids, alpha-linolenic and linoleic acids. The method involves feeding LCP? supplemented, nutrient-enriched formulas for an extended feeding regimen, typically until at least 3 months corrected age (CA), preferably to 6 or even 12 months CA. The neurological developments, for example, visual development, motor development and language development were enhanced without findings of anthropometric growth faltering or inhibition.
Feed products comprising rumen protected sources of polyunsaturated fatty acids in order to produce enriched milk are disclosed in for example JP 8336360, US 5,932,257, WO 00/44239, WO 02/00028, CA 2 524 451, US 2004/0058003 and US 5,985,348 as well as Sharon T. Franklin et al., Dietary Marine Algae (Schizochytrium sp.) Increases Concentrations of Conjugated Linoleic, Docosahexaenoic and Transvaccenic Acids in Milk of Dairy Cows, Nutrient Metabolism, XP000879209, page 2048-2054, 1999.
Feed additives for ruminants comprising tablets or granules of a mixture of active ingredients, chitosan and protective material are disclosed in United States Patent No. 4,533,557. Nutritive and medical compositions for ruminants comprising active substances coated with a composition based on chitosan salt and fatty matter are disclosed in EP 0 588 707.

### SUMMARY OF THE INVENTION

The invention provides a feed additive to be fed to cattle to achieve a milk product that contains elevated levels of polyunsaturated LC n-3 FA, particularly DHA, EPA, and/or DPA. It is intended that consumption of this LC n-3 FA-enriched milk will promote human health and prevent disease.

The milk product comprises at least 0.3% DHA as a percent of total milk fatty acids (FA). In a preferred embodiment, the milk comprises at least 0.35%, more preferably 0.4% DHA as a percent of total milk FA.

In a preferred aspect, a milk product comprising at least 0.3% DHA and at least 0.1% EPA as a percent of total milk FA is provided.

In a preferred embodiment, dairy product comprises a product selected from the group consisting of milk, yogurt, cheese, cream, spread, butter, frozen dessert, and baby formula.

A milk product comprising therapeutic levels of DHA & EPA & DPA is also provided.

A method of optaining a dairy product as defined in the claims is provided.

In an aspect of the invention, a method of obtaining a dairy product comprises feeding to a dairy cow a composition comprising fishmeal having at least 1 g DHA/100 g fishmeal, preferably 1.2 g DHA/ 100g fishmeal.

A method of meeting the human need for LC n-3 FA (including for the prevention and treatment of cardiovascular disease) through consumption of milk products having at least 0.3% DHA as a percent of total milk FA is also provided.

A feed product for dairy cows comprising a source of PUFA and a protectant as defined in the claims is provided.

The feed product uses fishmeal as a source of PUFA and the fishmeal preferably comprises at least 1 g DHA/ 100g fishmeal, more preferably at least 1.2 g DHA/100g fishmeal.

In another preferred embodiment, fish oil is used as a source of PUFA wherein the fish oil comprises at least 12 g DHA/ 100g fish oil.

The feed product includes a protectant selected from the group consisting of chitin, chitosan.

In another aspect, a food product comprising a DHA and/or EPA and/or DPA enriched dairy product is provided.

The food product may take many forms. It may be a dairy product including milk, yogurt, frozen desserts, etc. Alternatively, it may comprise a soup, sauce, gravy, prepared vegetables, and frozen meals.

### BRIEF DESCRIPTION OF THE DRAWINGS

These and other features of the invention will become more apparent from the following description in which reference is made to the appended drawings wherein:

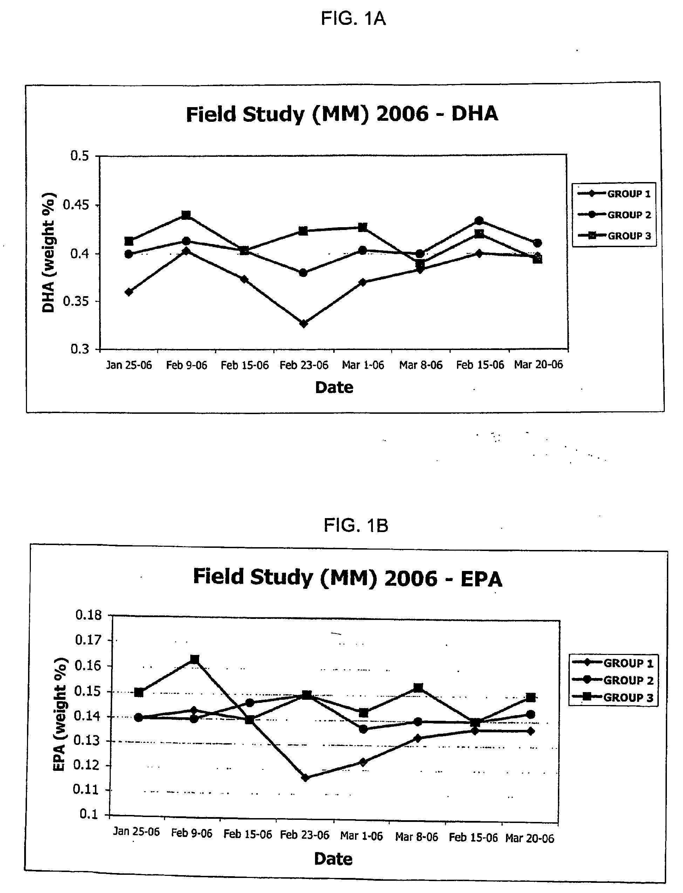

FIGURE 1A illustrates graphically the effect of various protectants on DHA levels in milk; and
FIGURE 1B illustrates graphically the effect of various protectants on EPA levels in milk.

### DETAILED DESCRIPTION

Milk is an excellent vehicle by which to achieve widespread human intake of essential nutrients. Not only is milk consumed directly by all age groups, but is widely used in the preparation of other dishes. Preliminary work done at the University of Guelph demonstrated that when DHA is provided to cows in a patented diet, the DHA is incorporated into the milk and the problem of an unpleasant flavour of the milk is completely eliminated. As described in United States Patent No. 5,932,257, when dairy cattle are fed a feed additive comprising a source of DHA and an inhibitor of microbial degradation of DHA in the rumen comprising feathermeal, the expressed milk from the dairy cattle is enriched for DHA. While this method provides a source of milk that contains DHA, there remained a need for improved methods of getting higher levels of DHA and other healthy LC n-3 FAs into milk.

Milk fat is synthesized either from fatty acids taken up from the blood (60%) or synthesized de novo in the mammary gland (40%). LC n-3 FA are not synthesized by ruminant tissues, so their concentration in milk is dependent on how much they ingest and on the amount that flows out of the rumen and is absorbed from the digestive tract. The rumen, however, is a site of intense microbial lipid metabolism. Under normal conditions, LC n-3 FA like DHA, are hydrogenated by rumen bacteria via a process called biohydrogenation. This results in a very low level of LC n-3 FA reaching the small intestine where they can be absorbed.

During the various steps of rumen biohydrogenation (hydrolysis, isomerization and hydrogenation), numerous intermediate compounds are produced. Research has shown that some isomers cause serious milk fat depressions even when small amounts (10 grams or less) reach the small intestine. The presence of certain FA in the mammary gland is thought to interfere with enzymes (Acetyl CoA carboxylase, fatty acid synthase, stearolyl CoA desaturase) involved in de novo fatty acid synthesis. Thus, the problem of getting milk enriched for LC n-3 FA without decreasing the total milk fat levels is not easily solved.

The presence of LC n-3 FA in milk is affected by two main factors: levels of LC n-3 FA in the diet and the presence of other components in the diet that help the LC n-3 FA avoid rumen biohydrogenation.

The present invention addresses each of these factors to synergistically obtain an improved product.

The first category includes factors that affect the amount of dietary LC n-3 FA offered to dairy cows. This involves the formulation of ruminant diets using LC n-3 FA-containing ingredients in sufficient quantities to achieve the desired level in milk. The second category includes any factors that affect the protection of dietary LC n-3 FA from rumen biohydrogenation, allowing greater transfer of intact LC n-3 FA to the small intestine and ultimately the mammary gland for inclusion in milk.

A preferred source of LC n-3 FA for use in a feedstock of the invention is fishmeal. The form in which LC n-3 FA are present in the fishmeal is key. Lipid fraction analysis of fishmeals revealed that the form of LC n-3 FA may vary significantly in different sources. An analysis of these samples is shown in Tables 1A, 1B, and 1C. In fishmeal the LC n-3 FA are present in a variety of forms: free fatty acids (FFA), triglycerides (TG) and phospholipids (PL). In the feedstock of the present invention, the fishmeal comprises at least 7% DHA in the total FA. The majority of the DHA is preferably associated with the phospholipid fraction. Preferred sources of LC n-3 FA comprise at least 1g DHA per 100g fishmeal. More preferred sources comprise at least 1.2 g DHA/100g fishmeal. Fishmeal is one preferred source of DHA and other LC n-3 FA, however other sources such as fish oil, algae oil, and algae may also be used. Indeed, when 60g/head/day of fish oil (12 g DHA/100g fish oil) was included in the diet of a milking herd the fishmeal was decreased at least 50% from normal levels without decreasing milk DHA below 0.3 % of milk fatty acids showing that fish oil LC n-3 FA acids can be substituted for fishmeal LC n-3 FA.

The second component that affects the levels of LC n-3 FA transferred from the diet to milk is a protectant. As used herein, the term "protectant" refers to any agent that protects dietary LC n-3 FA in the digestive tract and enhances uptake so that the dietary LC n-3 FA is transferred to an animal food product for human consumption.

In a further aspect of the invention, the protectant is chitin or chitosan. The chitin or chitosan is typically used in the feed in an amount ranging from about 0.25% to 2.0% on a dry weight basis. The results of an exemplary study entailing use of chitosan are shown in Figure 1. In addition, in a study using chitin, a rumen-fistulated cow was given a dose of a mixture of 120g chitin and 480g fish oil containing 24g DHA directly into the rumen. In the subsequent 2 days the arterial blood level of LC n-3 FA (DHA, EPA, plus DPA) were elevated by 1.02 to 1.51 % of total venous plasma fatty acids. These results demonstrate that chitosan can act as an effective protectant and that its use as a protectant leads to significantly elevated levels of milk DHA, EPA and DPA. and that chitin also can act as an effective protectant to yield an increased level of blood precursors of milk LC n-3 FA.

The invention also provides a method of producing milk having elevated levels of LC n-3 FA, particularly DHA, DPA, and EPA. The method comprises feeding a dairy cow a dietary source of LC n-3 FA. The LC n-3 FA may be derived from various sources. In a preferred embodiment the LC n-3 FA source is fishmeal comprising at least 7% DHA in total FA. DHA is used as a reference point to predict total Lc n-3 FA levels. Preferred fishmeal for use in the invention comprises at least 1.2 grams DHA per 100 grams fishmeal. Animals are fed between about 0.8 and 1.2 kilograms of the fishmeal per day. By using the preferred source of fishmeal the total amount of dietary fishmeal can be reduced below standard levels and high levels of LC n-3 FA in the milk can still be attained without a reduction in the total milk fat. The LC n-3 FA is combined with the protectant chitin, or chitosan. Animals are fed a nutritionally balanced ration which includes a feedstock or supplement comprising; i) about 0.5 to 10% LC n-3 FA , more preferably 2-5%; and ii) a protectant selected from the group consisting of chitin, and chitosan.

The feed supplement of the invention may be used either as a top dressing or as part of a total mixed ration. Good results for cows were found by feeding a total of about 1 to 5 kg preferably about 1.5 to 3.5 kg, more preferably about 2kg, of a feed supplement per animal per day. Additional components such as proteins, vitamins, minerals, buffers, ionophores and combinations thereof may be included in the feed supplement. The feed supplement of the present invention may vary in any one or all of the components described above. Further, one or more of the components may be absent and other components may be present in the feed supplement. In an embodiment of the present invention, the analysis for any one or all of the components may vary by about 20%, preferably less than 10%, more preferably less than 5% of the totals listed above.

The cows are typically fed the supplement for about 4 to 12 weeks (during the transition from a conventional feeding program to a LC n-3 FA feeding program) to achieve levels of DHA in excess of 0.3 % as a percent of total milk fat. The timing may vary from herd to herd. There may be an initial drop in milk fat content followed by a recovery. Particularly good results have been achieved by 10 weeks.

The invention also encompasses animal products such as milk and processed products, such as yogurt, ice cream, cheese and butter, produced by the method of the present invention that exhibit enhanced levels of LC n-3 FA including DHA, EPA and DPA. The present invention provides, for the first time a milk containing elevated levels of DHA plus EPA plus DPA. The milk of the invention comprises at least 0.3% DHA, preferably at least 0.35% DHA, more preferably at least 0.4% as a percent of total milk FA. A preferred milk also includes at least 0.1 % EPA and 0.1% DPA as a percent of total milk FA. Although DHA and other PUFAs are notoriously subject to oxidative degradation, the levels are very stable when secreted in milk. Milk, according to the invention, withstands ultra high temperature pasteurization and also has a subsequent shelf life in excess of 21 days. While the first obvious commercial product is fluid milk, other products are also encompassed. The milk, milk solids, cream and milk fat enriched in LC n-3 FA can also be ingredients for other food products. The present invention demonstrates for the first time that LC n-3 FA levels achieved in milk products by the methods of the invention remain stable for extended periods of time (i.e. greater than months). The levels remain stable even when the milk product is further processed such as by high temperature, for example, pasteurization or natural cooking. The milk product can be incorporated into processed foods such as cream, butter, ice cream, cheese, yogurt, soups, sauces, spreads, etc. Prepared foods such as packaged, potatoes or pasta and sauce products are also encompassed. This includes dried products. LC n-3 FA-enhanced milk products of the invention are particularly useful for inclusion in infant nutritional formulations.

In addition to enhancing the levels of LC n-3 FA in animal-derived products, the feed supplement of the invention provides health benefits to livestock as compared to standard feeds.

In addition, the food products derived from animals consuming the feed will be a source of the LC n-3 FA that are essential in the diet of humans and thereby provide health benefits to humans.

Dairy farmers are economically compensated (in part) based on the percent of fat in milk. A disadvantage of previous feeds containing PUFA is that they can cause a decrease in the percentage of total milk fat (as much as from 3.8 down to 2.8%), with no recovery. The synergistic compositions and methods of the present invention demonstrate that after an initial milk fat depression that occurs in some cases, the original milk fat level can be completely recovered in time (usually within 10 weeks). The present invention provides, for the first time, a feedstock and a method of feeding dairy cows whereby LC n-3 FA are fed to the animal and transferred to the milk without decreasing the percentage of fat in the resultant milk.

The above disclosure generally describes the present invention. It is believed that one of ordinary skill in the art can, using the preceding description, make and use the compositions and practice the methods of the present invention. A more complete understanding can be obtained by reference to the following specific examples. These examples are described solely to illustrate preferred embodiments of the present invention

### EXAMPLES

Although specific terms have been used in these examples, such terms are intended in a descriptive sense and not for purposes of limitation. Methods of molecular biology, biochemistry and chemistry referred to but not explicitly described in the disclosure and these examples are reported in the scientific literature and are well known to those skilled in the art.

### Example 1. Exemplary LC n-3 FA Dairy Supplement not according to the invention

The following ingredients are combined to provide a feed supplement that enhances the levels of LC n-3 FA in milk derived from dairy cows fed the supplement.

### Example Formula: LC n-3 FA Dairy Supplement

| | |
|---|---|
| Fish meal | 550 |
| Feather meal | 138 |
| Soybean meal | 110 |
| Soy hulls | 42 |
| Wheat shorts | 40 |
| Macro min/salt | 110 |
| Trace min/vit | 10 |
| | 1000 kg |

Dairy cows typically receive a daily dose of 2 kg of the supplement. Total dose may be altered for other animals.

### Example 2. The use of reduced amounts of fishmeal and feathermeal not according to the invention

Fishmeal & feather meal were fed to dairy cows at lower levels (0.9 kg & 0.225 kg/animal/day respectively) than the previously standard amounts. Unexpectedly, significant amounts of DHA were achieved in the milk. [0059] Two herds (C, M) were fed these levels for five months. The average milk DHA levels (expressed as DHA as %/g of milk fatty acids) in 11 samples for Herd 'C' is 0.30% and for Herd 'M' is 0.33%. The results demonstrate that significant levels of DHA in milk can be achieved using reduced amounts. In view of the large dietary consumption of cows, this represents very significant cost savings.

### Example 3. Use of chitosan as a protectant

Chitosan was used as a protectant in a whole herd field trial. The milk DHA level (expressed as % DHA as a percent of milk FA) was 0.46%. These results demonstrate that chitosan is a very effective protectant that enhances the transfer of dietary LC n-3 FA to milk.

The present invention has been described with regard to one or more embodiments.

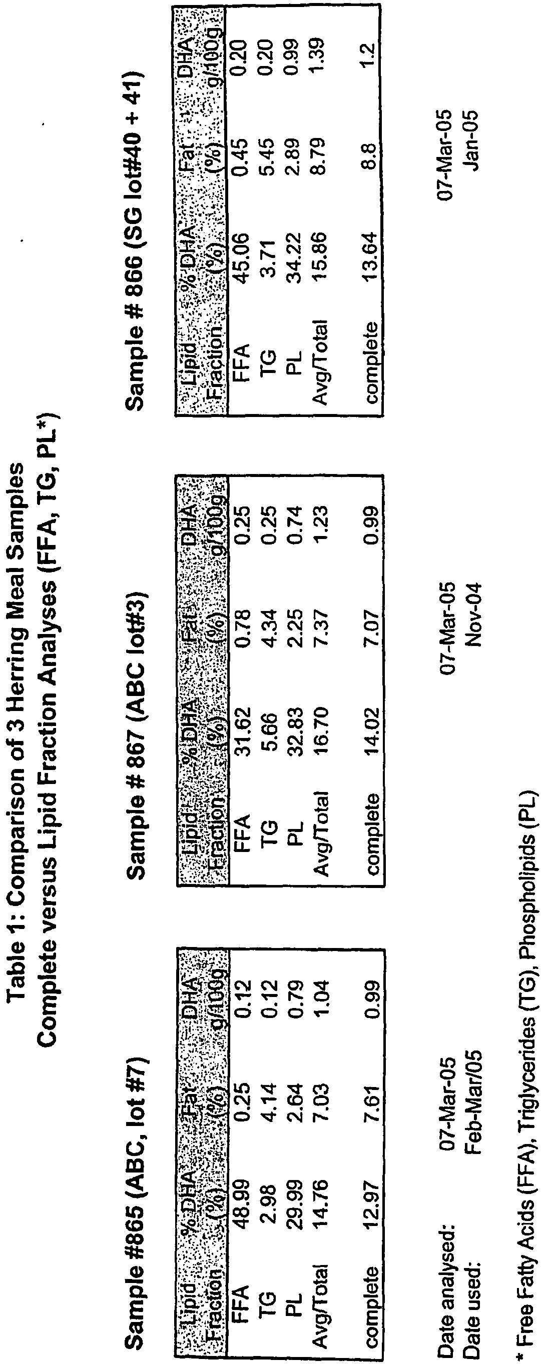

## Claims

1. A feed product for dairy cows comprising a source of LC n-3FA and a protectant, wherein the source of LC n-3 FA is selected from the group consisting of fishmeal, fish oil, algae oil and algae and the protectant is selected from chitin and chitosan.

2. A feed product according to claim 1, wherein the fishmeal comprises at least 1.2 g DHA/100g fishmeal.

3. A feed product according to claim 1 wherein the fish oil comprises at least 12 g DHA/100 g fish oil.

4. The feed product according to claim 1, wherein the protectant is present in an amount ranging from about 0.25% to 2.0% by weight.

5. A method of obtaining a dairy product comprising at least 0,3% DHA and at least 0.1% EPA as a percent of total milk fatty acids, comprising feeding to a deary cow a feed product as defined in claim 1.

6. The method according to claim 5, wherein the fishmeal comprises at least 1.2 g DHA/100g fishmeal.

7. The method according to claim 5, wherein the fish oil comprises at least 12 g DHA/100 g fish oil.

8. The method according to claim 5, wherein the protectant is present in an amount ranging from about 0.25% to 2.0% by weight.

9. The method according to claim 5, wherein the feed product is fed to the cow in an amount of about 1 to 5 kg per day, preferably about 1.5 to 3.5 kg per day, and more preferably about 2 kg per day.

## Patentansprüche

1. Futterprodukt für Milchkühe, umfassend eine Quelle für langkettige n-3-Fettsäuren und einen Schutzstoff, wobei die Quelle für langkettige n-3-Fettsäuren aus der Gruppe ausgewählt ist, die aus Fischmehl, Fischöl, Algenöl und Algen besteht, und der Schutzstoff aus Chitin und Chitosan ausgewählt ist.

2. Futterprodukt gemäß Anspruch 1, wobei das Fischmehl wenigstens 1,2 g DHA / 100 g Fischmehl umfasst.

3. Futterprodukt gemäß Anspruch 1, wobei das Fischöl wenigstens 12 g DHA / 100 g Fischöl umfasst.

4. Futterprodukt gemäß Anspruch 1, wobei der Schutzstoff in einer Menge vorhanden ist, die im Bereich von circa 0,25 Gewichtsprozent bis 2,0 Gewichtsprozent liegt.

5. Verfahren zum Gewinnen eines Milchproduktes, das wenigstens 0,3% DHA und wenigstens 0,1% EPA als einen Prozentanteil der gesamten Milchfettsäuren enthält, umfassend ein Füttern eines Futterproduktes gemäß Anspruch 1 an eine Milchkuh.

6. Verfahren gemäß Anspruch 5, wobei das Fischmehl wenigstens 1,2 g DHA / 100 g Fischmehl umfasst.

7. Verfahren gemäß Anspruch 5, wobei das Fischöl wenigstens 12 g DHA / 100 g Fischöl umfasst.

8. Verfahren gemäß Anspruch 5, wobei der Schutzstoff in einer Menge vorhanden ist, die im Bereich von circa 0,25 Gewichtsprozent bis 2,0 Gewichtsprozent liegt.

9. Verfahren gemäß Anspruch 5, wobei das Futterprodukt in einer Menge von circa 1 bis 5 kg pro Tag, bevorzugt circa 1,5 bis 3,5 kg pro Tag und weiter bevorzugt circa 2 kg pro Tag an die Kuh verfüttert wird.

## Revendications

1. Produit alimentaire pour vaches laitières comprenant une source de LC n-3FA et un agent protecteur, dans lequel la source de LC n-3FA est sélectionnée à partir du groupe se composant de la farine de poisson, de l'huile de poisson, de l'huile d'algues, et des algues et en ce que l'agent protecteur est sélectionné à partir de la chitine et du chitosane.

2. Produit alimentaire selon la revendication 1, dans lequel la farine de poisson comprend au moins 1,2 g de DHA/100 g de farine de poisson.

3. Produit alimentaire selon la revendication 1, dans lequel l'huile de poisson comprend au moins 12 g de DHA/100 g d'huile de poisson.

4. Produit alimentaire selon la revendication 1, dans lequel l'agent protecteur est présent dans une quantité s'étendant d'environ 0,25 % à 2,0 % du poids.

5. Méthode d'obtention d'un produit laitier comprenant au moins 0,3 % de DHA et au moins 0,1 % d'EPA en tant qu'un pourcent des acides gras totaux de lait, comprenant l'alimentation destinée à une vache laitière d'un produit alimentaire selon la définition de la revendication 1.

6. Méthode selon la revendication 5, dans laquelle la farine de poisson comprend au moins 1,2 g de DHA/100 g de farine de poisson.

7. Méthode selon la revendication 5, dans laquelle l'huile de poisson comprend au moins 12 g de DHA/100 g d'huile de poisson.

8. Méthode selon la revendication 5, dans laquelle l'agent protecteur est présent dans une quantité s'étendant d'environ 0,25 % à 2,0 % du poids.

9. Méthode selon la revendication 5, dans la quelle le produit alimentaire est donné en alimentation à la vache dans une quantité d'environ 1 à 5 kg par jour, de manière préférable d'environ 1,5 à 3,5 kg par jour, et de manière plus préférable d'environ 2 kg par jour.
